# EUROPEAN PATENT APPLICATION

(11) **EP 1 658 816 A1**
(43) Date of publication of application: **24.05.2006**
(21) Application number: 05023593.6
(22) Date of filing: 28.10.2005
(51) Int. Cl.: A61B 17/86

(54) **Surgical screw with spontaneous loosening preventive blocking system**

(30) Priority: 19.11.2004 IT RE20040143
(71) Applicant: Rovesti, Gian Luca, 42025 Cavriago (Reggio Emilia) (IT)
(72) Inventor: Rovesti, Gian Luca, 42025 Cavriago (Reggio Emilia) (IT)
(74) Representative: Lecce, Giovanni

(57) **Abstract**

A surgical screw featuring a spontaneous loosening preventive blocking system comprises a threaded stem (1) provided with a head (2); at least one inclined hole (3) going through said head (2) coming out on one side of said threaded stem (1); and a stainless metal thread or nail (9) for surgical use inserted in said inclined hole (3) and getting into insertion and engagement relationship with a fractured bone (8) to be compounded.

## Description

### BACKGROUND OF THE INVENTION

### Field of the invention

The present invention relates to a surgery screw featuring a spontaneous loosening preventive blocking system.

More particularly the present invention relates to a surgical screw featuring a spontaneous loosening preventive blocking system particularly suitable to be used in surgical human and/or veterinarian orthopaedics.

### Description of the prior art

It is known that in orthopaedic surgery plates and screws are widely used. Under ideal conditions, the problem of loosening screws, particularly during the post-surgery period, is generally not considered among the highly frequent complications.

Under non ideal conditions, e.g. in case of particularly small diameter screws, with a state of fractured bones characterized by diminished solidity, or in case of particularly strong mechanical stress, the problem of screws spontaneous loosening becomes significantly remarkable, with particular reference to potentially disastrous consequences which might have an effect on the implant's reliability.

The same problem of spontaneous loosening becomes highly significant in case surgery is realized on young people or animals, with reduced bone consistency, or in case the implant positioning is done under mechanically unfavourable conditions.

### SUMMARY OF THE INVENTION

Object of the present invention is to avoid the above reported inconveniences.

More particularly, object of the present invention is to provide a surgical screw that has no spontaneous loosening problems.

A further object of the present invention is to provide a surgical screw provided with a spontaneous loosening preventive blocking system.

According to the present invention the above and further objects, which will be apparent from the following description, will be achieved by means of a surgical screw comprising a threaded stem featuring a head provided with at least one inclined through hole coming out of one side of said stem, and a stainless metal thread or surgical nail inserted in said inclined hole and getting into insertion and engagement relationship with a fractured bone to be compounded.

The screw, which might belong to any traditional type of orthopaedics surgery screws both for human and veterinarian, comprises a head provided with one or more holes wherein pieces of thread or stainless metallic nails can be inserted, destined to surgical use, getting into engagement relationship, on one side, with the fractured bones to be compounded and, on the other side, with the screws' head, by shaping on their profile or by direct engagement.

The advantages achieved by the surgical screw of the present invention essentially derive from the fact that the one or more threads, or the one or more metallic nails, being inserted into one or more inclined holes into the screw's head, which are introduced into the fractured bones to be compounded and engage with the head itself, represent a bond making impossible any rotational movement of the screws, avoiding the spontaneous loosening.

Further and remarkable advantages derive from the fact that the blocking device of the present invention, besides being effective, is very economic and characterized by an easy and quick implementation, it does not require any substantial modification to current surgery techniques, and relative tools, and can be easily adopted in applications with or without the use of plates.

In summery, the fundamental and innovative characteristics of the surgical screw with rotation blocking system of the present invention are:
- high resilience to spontaneous loosening (particularly during the post surgery period),
- compatibility to conventional application implants of current orthopaedics surgery, both humans and veterinarian,
- compatibility of use with the commonly adopted tools of traditional insertion (the use of specific tools is avoided)
- ease of use of the implementation technique (it shortens surgical time)
- low cost

### BRIEF DESCRIPTION OF THE DRAWINGS

The invention is described in details, with reference to some embodiments given with the sole and non limitative intention and with reference to the appended drawings where:
fig. 1 represents a schematic section view of a screw of the present invention, engaged on an osteosynthesis with plate, of a fractured bone, and
fig. 2 represents schematic views of some embodiments of heads featuring one or more inclined through holes, according to the present invention, with different types of screwing.

### DESCRIPTION OF THE PREFERRED EMBODIMENT

With reference to the figures, the screw for surgical application of the present invention, suitable for both human and veterinarian orthopaedics implementations, comprises a threaded stem 1 provided with a head 2 featuring at least one inclined hole 3.

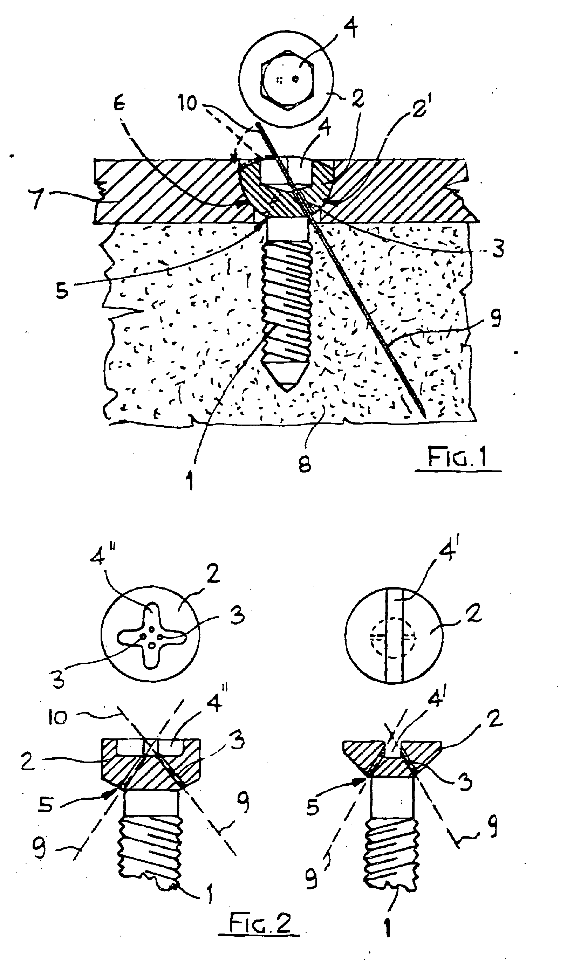

Said at least one inclined hole 3 goes through head 2, starting from above or, preferably, from inside of any type of screwing means of engagement 4 and coming out inclined on one side of the threaded stem 1, substantially in correspondence to the point of connection 5 with the lower part of the head itself. In the example of figure 1, head 2 of the screw represented with a screwing means of engagement 4, carved hexagon-like and with inferior part 2', whose shape is like a convex inverted hemispheric truncated-cone facing the stem. Said example is typical of a screw, particularly suitable for traditional orthopaedics surgery applications using commonly available tools for inserting and locking operations and with the inferior portion 2' whose convex hemispherical truncated-cone shape allows and easy coupling, even though not perpendicular, featuring concave seats 6 for the blocking plates 7. Same functional principles of the present invention relative to blocking of spontaneous loosening, is easily applicable to screws provided with different screwing engagement means, such as transversal notches 4', star-like notches 4", o similar, made on same or different head configurations, such truncated-cone one with or without abutting plane, cylindrical or of any other type.

On the example reported, said one or more inclined holes 3, start from the blind base of the hexagonal step 4 and they go out inclined in correspondence to said point of connection 5, between the inferior part of head 2 and the superior coupling of threaded stem 1.

Surgical method for the execution of the intervention procedure is usually adopted and followed without variations, until the screws' locking on bone 8 is achieved. (in the example reported, by means of proper seat 6 of plate 7).

Once screwing has been completed, a thread or a surgical nail in stainless metal 9 (e.g Kirschner) is inserted into holes 3 of at least one of the screws for each side of the osteothomy or fracture line, and after going through head 2, it comes out inclined from one side of threaded stem 1 and transversely gets into internal part 8 of the bone to be compounded, along the appropriate length, as a function of the fracture itself. In particular, for example and preferably, the length of the thread or nail 9 which has been inserted into the bone is at least equal or close to the thickness of the bone itself.

In case of use of a thread, once inserted, it can be cut leaving an external piece 10 (e.g. few mm) to be bent by means of pliers, in order to create a bend to be shaped on the screw's head; diversely, it simply can be cut on the external edge of the head itself.

In case of use of a nail, it is preferably inserted until its superior part gets into position relationship with said engagement means 4, 4', 4".

Remaining surgical issues are carried out as usual.

The screws according to the present invention can be used, in the same way and for the same purposes, weather or not plates 7 are present.

It is further important to point out that the screw can be provided with three through holes 3, inclined and with radial orientation. This in order to give the operator the choice between different angles of inserting the thread or nail 9, both to assure the position into the bone, and, further, to avoid any alignment to plates 7, which would became an obstacle to the insertion.

Another positive condition comes from the fact that numerous holes make possible the use of multiple threads or nails 9 per screw, making the implants more stable.

In conclusion, the screws object of the present invention can be easily used both for human and veterinarian orthopaedics surgical implants both stable and provisional or temporary.

While the present invention has been described and illustrated according to some embodiments, given with the sole intention to supply an example and not to limit, it will became apparent to a person skilled in the art that various modifications to shapes, particular parts and types of rotation and coupling means can be conceived within the scope of protection of the following claims.

## Claims

1. A surgical screw featuring a spontaneous loosening preventive blocking system **characterized in that** it comprises a threaded stem (1) provided with a head (2); at least one inclined hole (3) going through said head (2) coming out on one side of said threaded stem (1); and a stainless metal thread or nail (9) for surgical use inserted in said inclined hole (3) and getting into insertion and engagement relationship with a fractured bone (8) to be compounded.

2. The surgical screw as claimed in claim 1, **characterized in that** head (2) is provided with a screwing engagement means (4) as a caved hexagon, whose inferior portion (2') straight or convex hemispheric, faces toward stem (1).

3. A surgical screw according to claim 1, **characterized in that** head (2) is provided with engagement means for screwing with transversal notches (4') or star-like notches (4").

4. A surgical screw according to any of the previous claims, **characterized in that** said at least one inclined hole (3) goes through head (2) starting from any screwing engagement means (4, 4', 4") and comes out inclined from a side of the threaded stem (1), close or in correspondence to the connection point (5) with the head itself.

5. The surgical screw according to any of the previous claims from 1 to 3, **characterized in that** said at least one inclined hole (3) starts from any inferior point of the head (2) and comes out inclined on one side of said threaded stem (1), close or in correspondence to its connection point (5) with the inferior part of the head itself.

6. The surgical screw according to any of the previous claims, **characterized in that** the stainless metal thread or nail (9) for surgical use, going through said at least one inclined hole (3) of head (2), gets into insertion and inclined engagement relationship with a fractured bone (8) to be compounded, along a length depending on the fracture extension.

7. The surgical screw according to claim 6, **characterized in that** the stainless metal thread or nail (9) for surgical use, going through said at least one inclined hole (3) of head (2), gets into insertion and inclined engagement with fractured bones (8) to be compounded, along a length depending on the thickness of the bone itself.

8. The surgical screw according to any of the previous claims, **characterized in that** head (2) comprises one or more inclined through holes (3) oriented with radial orientation, and through at least one of said inclined holes with radial orientation (3), the stainless metal thread or nail goes through.

9. The surgical screw according to any of the previous claims, **characterized in that** the stainless metal thread or nail (9) for surgical use is cut leaving an external piece (10) shaped as a bend on the head's (2) profile.

10. The surgical screw according to any of the previous claims from 1 to 8, **characterized in that** the stainless metal thread (9) for surgical use is cut in correspondence to the external edge of head (2).

11. The surgical screw according to any of the previous claims from 1 to 8, **characterized in that** the superior part of the stainless metal nail (9) for surgical use gets into positioning relationship with the engagement means (4, 4', 4").

12. The surgical screw according to any of the previous claims, **characterized in that** said head (2) has a cylindrical configuration, whose inferior convex hemispherical or truncated cone part faces towards the stem.
